# EUROPEAN PATENT SPECIFICATION

(11) **EP 2 603 141 B1**
(45) Date of publication and mention of the grant of the patent: **26.07.2017**
(21) Application number: 11749140.7
(22) Date of filing: 10.08.2011
(51) Int. Cl.: A61B 5/103, A61B 5/107

(54) **DEVICE AND SYSTEM FOR EVALUATING CONDITION OF SKIN, SCALP AND HAIR**
VORRICHTUNG UND SYSTEM ZUR BEURTEILUNG DES ZUSTANDES VON HAUT, KOPFHAUT UND HAAR
DISPOSITIF ET SYSTÈME PERMETTANT D'ÉVALUER L'ÉTAT DE LA PEAU, DU CUIR CHEVELU ET DES CHEVEUX

(30) Priority: 13.08.2010 US 855727
(43) Date of publication of application: 19.06.2013
(73) Proprietor: Unilever PLC, London EC4Y 0DY (GB); Unilever N.V., 3013 AL Rotterdam (NL)
(72) Inventor: NICHOL, Jamie, Gordon, Arlington, MA 02476 (US); CICHOWLAS, Bruce, Framingham, MA 01701 (US)
(74) Representative: Fijnvandraat, Arnoldus
(86) International application number: PCT/EP2011/063744
(87) International publication number: WO 2012/020043

(56) References cited:
- US-A1- 2002 065 452
- US-A1- 2004 171 962
- US-A1- 2007 185 392

## Description

The present invention relates to an apparatus, method and computer program product for evaluating the condition of the skin, scalp and hair. In particular, the invention relates to aggregating and analyzing data collected by a user and producing a recommendation for the user to determine and possibly improve the condition of her skin, scalp and/or hair.

Consumers generally desire to know the condition of their skin, scalp and hair, what are the most efficacious products to choose from, and how effective such products are over time. One solution to this problem is an apparatus, method and computer program product designed for the rapid analysis of the skin, scalp and hair that can be conveniently utilized by a user that can diagnose his or her skin, scalp and hair condition and lastly can monitor its improvement over time. Advantageously a selection of products can be suggested to the user for treating the conditions disclosed.

Many devices, systems and methods suitable for disclosing skin, scalp and hair deficiency amenable to treatment are known. However, the majority of these suffer from significant drawbacks of reliability, ease of use and convenience with consumers that can be employed in shops or in their own homes.

PCT publication no. WO08064120A published on May 29, 2008 discloses a method and system for a person to objectively screen himself or herself for increased skin cancer risks using specific skin image parameters in conjunction with a digital photograph and a computer.

U.S. Patent No. 5938593 issued to Ouellette on August 17, 1999 describes a skin condition analyzer with selectable modes of operation that includes a probe apparatus for generating a skin condition signal representative of the moisture content of the skin and a processor that adjusts the skin condition signal in accordance with environmental components, such as temperature and humidity, to arrive at an overall skin condition signal.

U.S. Patent publication no. 2002065452A to Bazin et al. published on May 30, 2002 discloses a process for diagnosing, based on scanned information, one or more conditions of the skin and/or one or more features a product applied to the skin. The scanned image data may be transferred from a first computer associated with the scanner to a second computer at a remote location. An analysis of the skin condition is conducted based on the image data. The process further includes determining a diagnosis and may also involve determining a recommendation of treatment for the condition.

US 2007/185392 A1 discloses a device for providing an indication of moisture present in skin of a user. A sensor circuit is used to generate a signal having a frequency that corresponds to moisture content at a skin measurement location. A binary counter is connected to the sensor circuit and a microcontroller measures a pulse count from the binary counter which is stored in memory as a numerical skin moisture value. US 2004/0171962 A1 describes an apparatus and method to evaluate hydration of skin or mucous membranes.
Surprisingly, it has now been found that sensors capable of sensing a property of the skin, scalp and hair and producing an analog signal related to that property can be employed either individually, or in any combination to analyze the skin, scalp and/or hair whereby the analog signal(s) produced is/are converted into an audio signal, and relayed to a PC or Apple class computer, or mobile device or an equivalent thereof via a suitable serial interface such as a USB interface, Bluetooth connection or serial port connection and the like. A software application such as Flash® (Adobe), HTML5 (open source) or C++ (Microsoft or open source) or any other suitable application serves to transform the raw data into output that is directly correlated with the user's skin, scalp or hair condition and optionally generates a report. In a preferred embodiment, the data is directed to a specific website via a suitable browser interface over the internet whereby a PC or Apple class computer or a mobile device enables the user to produce customized reports, obtain a recommended treatment regime suitable for treating the deficiency discovered, or view a selection of products for treating the condition disclosed.

The combination of the reliability of the audio signal transformation and its analysis coupled with flexibility of e.g. the USB interface or the like was unexpectedly found to provide the user with a powerful yet easy to use tool to manage their skin, scalp and hair treatment without complex equipment or special expertise beyond what the user is likely to own aside from the skin, scalp or hair sensor and its dedicated electronic circuitry.

In one aspect of the invention there is provided an apparatus for analyzing the condition of skin, scalp or hair of a user, including but not limited to:
a. a transducer device responsive to a property of the skin, scalp or hair providing an analog output related to the property;
b. a serial device for outputting a digital signal from an audio signal input;
c. wherein the audio signal is generated from the transducer analog output related to the property; and
d. wherein the serial device provides data packet transmission sufficient for an application to find both a point at which a new packet begins and an opportunity to interpret its received raw data signal.

In another aspect of the invention, there is provided a method for analyzing the condition of skin, scalp or hair of a user, including but not limited to the steps of:
a. contacting the skin, scalp or hair with a sensor coupled to a transducer device responsive to one or more properties of the skin, scalp or hair providing an analog output related to that property;
b. transmitting an audio signal from the transducer analog output to a serial device;
c. outputting a digital signal from the serial device; and
d. wherein the serial device provides data packet transmission sufficient for an application to find both a point at which a new packet begins and an opportunity to interpret its received raw data signal.

In a further aspect of the invention, there is provided a computer program product for analyzing the condition of skin, scalp or hair of a user, including but not limited to:
a. at least one computer-readable storage medium having computer-readable program instructions stored therein;
b. the computer readable program instructions including a program instruction for receiving digital data from a serial input device converted from audio data derived from a skin, scalp or hair analysis transducer analog device related to one or more properties of the skin, scalp or hair;
c. wherein the serial device provides data packet transmission sufficient for an application to find both a point at which a new packet begins and an opportunity to interpret its received raw data signal;
d. a program instruction for comparing the serial input device digital data to a database of data related to skin, scalp and hair condition;
e. a program instruction for creating a transfer protocol request from a transfer protocol client application executed on the computing device;
f. wherein the first transfer protocol request is directed to a first network location; and
g. wherein a client application at the first network location produces an output related to the serial input device digital data.

Having thus described embodiments of the invention in general terms, reference will now be made to the accompanying drawing, which is not necessarily drawn to scale, and wherein:

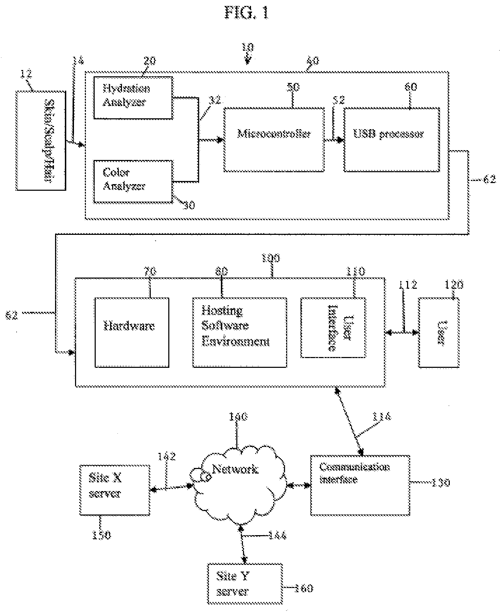

- Figure 1 is a schematic block diagram depicting a system for analysis of hair and skin according to an exemplary embodiment of the present invention.

In one aspect of the invention there is provided an apparatus for analyzing the condition of skin, scalp or hair of a user, including but not limited to:
a. a transducer device responsive to a property of the skin, scalp or hair providing an analog output related to the property;
b. a serial device for outputting a digital signal from an audio signal input;
c. wherein the audio signal is generated from the transducer analog output related to the property; and
d. wherein the serial device provides data packet transmission sufficient for an application to find both a point at which a new packet begins and an opportunity to interpret its received raw data signal.

Preferably the data packet transmission is interspersed by pauses; is interspersed by repetitive data sequences that do not appear in the serial device digital signal; or is interspersed by an escape sequence (which is useful in the instance where there is no unique pattern known not to occur in the serial device). Advantageously the property of the skin, scalp or hair the transducer is responsive to is selected from a mechanical property (such as elasticity, viscoelasticity, distensibility, indentability and the like by way of testing techniques including but not limited to ballistometry, pressure or suction resistance, tensile strength or frictional measurement, etc.), a moisture property (such as TEWL or transepidermal water loss measurement, or corneometry etc. by way of testing techniques including but not limited to hygrometry, capacitance, or conductance, etc.), a color property (by way of testing techniques including but not limited to reflectance or absorption photometry or spectrophotometry, etc.), a gloss property (such as shine by way of testing techniques including but not limited to reflectometry, etc.) or a combination thereof.

In a preferred embodiment the invention further includes:
a. a processor configured to receive the digital signal from the serial device;
b. an application (such as Flash® (Adobe), C++ (Microsoft or open source), HTML5 (open source), Symbion ® (Nokia) etc.) residing in the processor which interprets the digital signal;
c. wherein the application generates an output related to the digital signal on a user interface; and
d. wherein the output is displayed as a signal level, an image, a report, a local or remote data file or within a networked application script or a combination thereof.

Preferably the moisture property is measured by a hydration transducer including an external surface contactable against skin, scalp or hair including at least two adjacent metallic wires with their respective capacitance sensitive to differences in dielectric constant and the color property is measured by a color transducer including a plurality of light emitting diodes arranged for emitting light and also absorbing light reflected from the skin, scalp or hair area being evaluated.

Advantageously the inventive apparatus further includes at least one digital sensor responsive to a property of the skin, scalp or hair; preferably a digital camera. More preferably the serial device is selected from a USB processor, Bluetooth processor, ZigBee processor, Ethernet processor, PS/2 processor or a combination thereof. In a further preferred embodiment the apparatus also includes a communication interface connecting the user interface to a remote processor wherein the remote processor generates output related to the skin, scalp or hair condition of the user. Preferably the remote processor is connected to the communications interface over a network.

In another aspect of the invention there is provided a method for analyzing the condition of skin, scalp or hair of a user, including but not limited to the steps of:
a. contacting the skin, scalp or hair with a sensor coupled to a transducer device responsive to one or more properties of the skin, scalp or hair providing an analog output related to that property;
b. transmitting an audio signal from the transducer analog output to a serial device;
c. outputting a digital signal from the serial device; and
d. wherein the serial device provides data packet transmission sufficient for an application to find both a point at which a new packet begins and an opportunity to interpret its received raw data signal.

In a preferred embodiment the inventive method further includes:
a. transmitting the digital signal from the serial device to a processor;
b. interpreting the digital signal by a compatible application residing in the processor;
c. generating an output related to the digital signal; and
d. displaying the output as a signal level, an image, a report, a local or remote data file or within a networked application script or a combination thereof.

Advantageously a communication interface connects a user interface to a remote processor wherein the remote processor generates output related to the skin, scalp or hair condition of the user sampled by the transducer(s). More preferably the remote processor is connected to the communications interface over a network.

In a further aspect of the invention there is provided a computer program product for analyzing the condition of skin, scalp or hair of a user, including but not limited to:
a. at least one computer-readable storage medium having computer-readable program instructions stored therein;
b. the computer readable program instructions including a program instruction for receiving digital data from a serial input device converted from audio data derived from a skin, scalp or hair analysis transducer analog device related to one or more properties of the skin, scalp or hair;
c. wherein the serial device provides data packet transmission sufficient for an application to find both a point at which a new packet begins and an opportunity to interpret its received raw data signal;
d. a program instruction for comparing the serial input device digital data to a database of data related to skin, scalp and hair condition;
e. a program instruction for creating a transfer protocol request from a transfer protocol client application executed on the computing device;
f. wherein the first transfer protocol request is directed to a first network location; and
g. wherein a client application at the first network location produces an output related to the input device digital data.

In terms of signal processing, elements of the data handling used for the invention are known in the field. However its unique combination with the skin, scalp and/or hair transducers of the invention delivered exceptional and surprising results. This data handling approach may be used for any sort of USB or analogous serial device where a limited amount of data is being relayed to a CPU whether in a PC or Apple class computer or mobile device or the like.

It is essential to this invention that the skin, scalp and/or hair data be repetitive. It is also essential that missing short events in the data not cause a problem to the software application employed in its analysis such as Flash® or its equivalent. For instance, the serial number of the analysis device is one of the data elements transmitted that would be recognized as being constant or nearly so. If the serial number were to change for e.g. a quarter of a second to another value and then change back, the application software might or might not detect that change. However, if the serial number changed to another value and then stayed there for longer than about 0.5 seconds, the software would likely detect that change within half a second or so.

The following analogy will be useful to understand one aspect of the invention but should not be construed to limit it in any way. An observer sees an identifiable stream of data packets where the packets may each vary in their value. The sequence of the data packets is important and the final packet is the sum of all the other packets in the stream i.e. the checksum. It is important to note the sequence of numbers within each stream. Unfortunately, the sampling of the stream may be interrupted for some indeterminate length of time due to one or more causes. The data stream however continues although the individual data packets may each vary in value. However it remains important to note the sequence of data packets.

Uninterrupted analysis of each distinct stream of data packets and their checksum is straightforward and efficient. Interrupted analysis is a problem that must be solved lest the interrupted data packets be discarded resulting in inefficient data analysis. Algorithms are known that allow combining fragments of data streams but in general there can be inaccuracies in doing this, since one is combining sequences of numbers that didn't originate together.

The USB or an equivalent serial device furnishes the data values in the invention in an uninterrupted fashion. However, the computer's or mobile device's operating system (for example, Windows®, Apple® or other equivalent system) and its resident application (for example, Flash® (Adobe), HTML5 (open source) or C++ (Microsoft or open source) etc. or other equivalent application) can and often impairs the reliable and consistent sampling of the inputted data. One of the benefits of the invention was discovered to be the reliable and efficient analysis of the skin, scalp and hair data notwithstanding the impairment of data sampling described above.

FIG. 1 illustrates a preferred embodiment of the skin, scalp and/or hair analysis apparatus and method (10) of the present invention in schematic format. Since the output of suitable analyzers for analyzing the skin, scalp or hair (12) such as a hydration analyzer (20) and color analyzer (30) are usually analog, it was found useful to convert the analog output (32) to digital values that are stored in a suitable digital data storage device such as micro-controller (50). These digital values are then converted to an audio signal (52). Audio signal (52) is digitized by USB processor (60) to output digital signal (62). Audio signal (52) may include audio tones such as DTMF, MF4, CW, or the like.

The data relevant to the condition of the skin, scalp or hair is then extracted from the audio signal (52). Thus, data (32) is converted from analog to digital to analog to digital ("ADAD"). ADAD conversion was surprisingly found to be a key component to using off-the-shelf components to transfer skin, scalp or hair data in a way a PC or Apple class computer or a mobile device etc. recognizes and that the average user is already likely to own or have access to. Also depicted in FIG. 1 is a preferred embodiment where transducers (20 and 30), microcontroller (50) and USB processor (60) are contained in a compact, unitary device (40) that may be conveniently handled by a user.

The USB digital signal (62) is then processed by hardware (70) and hosting software environment (80) and input to user interface (110) within device (100). The software application employed such as Flash®, HTML5 or an equivalent can be hosted by either a browser or the operating system which is part of the hosting software environment (80). As discussed above, device (100) may include a PC or Apple or equivalent class computer or a mobile device which a user (120) may interact with via user interface (110). The user (120) may be the same or a different person whose hair, scalp or skin (12) has been analyzed. In a preferred embodiment user interface (110) is connected to a network (140) that is also connected to one or more site servers (150, 160). User (120) may request remote analysis of any or all of the data (14). A report or other output may then be generated remotely for display on the user interface (110), optionally including a recommendation for an appropriate product to treat the person's skin, scalp or hair that was analyzed by sensing device (40).

The USB processor (60) may be replaced with any serial protocol and compatible device that can digitize the audio signal (52) and input it into hardware (70) and hosting software environment (80) that is adapted to handle it. Various serial protocols are also useful in the invention and can include Bluetooth, ZigBee, Wi-Fi, Ethernet, PS/2 and the like in addition to USB. Advantageously, for ease of use, the transfer of bits of data should be accomplished without requiring any additional device drivers or hardware other than those already present with Flash® or an equivalent application already likely to be installed on a user's PC or Apple class computer or mobile device.

Alternatively the data on the skin, scalp or hair hydration or color could be transformed to appear as if it was residing on e.g. a USB mass storage device. However, this would be disadvantageous as it would require additional hardware and firmware and pose problems to merge easily into the widely available applications discussed above. This is in part due to security features within Flash® and similar applications and application environments governing the processing of data that involves internet and local files, particularly reading local files. However, Flash® for example, is designed to process sampled audio (microphone) data (starting in version 10.1), so by artificially generating microphone data, some amount of data can be entered into the Flash® application in spite of such security limitations.

In a preferred embodiment, Flash® and its Action script interpreter run at a fairly high level (compared, for instance, to most device drivers) therefore incurring a certain amount of inefficiency and lack of precision in sampling time. By design, the finest level of time interrupt in flash is that of "frame refresh" of the Flash® image on the screen of the user interface (110). While the Flash® application writer can set a desired number of frames per second for this quantity, it only serves as a hint to Flash® of what is desirable and the actual rate may vary from that or incur pauses. The USB device can't be sure that Flash® is keeping up with the frame rate or even listening at all at any point in time. Requesting a faster frame rate may give faster Flash® interrupts but also introduce unreliability and instability if Flash® is unable to keep up with the rate requested. In addition, other applications (e.g. Windows®) and user interactions with the screen (such as moving and resizing windows) can all reduce the opportunity for Flash® or an equivalent application to run smoothly.

Because of the above factors and the relatively low amount of skin, scalp or hair analysis data to be transmitted, it was surprisingly found that a scheme in which a serial device such as a USB device would repeat its transmission repeatedly, not knowing which data transmissions were actually processed would serve to improve reliability and accessibility to the user and a home computer or mobile device. The USB device would, in one embodiment, pause between its transmissions to give the Flash® application an opportunity to find the point at which a new packet begins and also an opportunity to interpret its received raw data signal (which simply consists of digital sampling of the audio signal at a regular interval of several thousand times per second). Other digital data transmission protocols for sampling signal (62) may be used and include but are not limited to repetitive data sequences separating the data packets that are not found in signal (62), an escape sequence where there is no unique pattern known not to occur in signal (62), and any other equivalent data transmission protocols that serve to identify the logical stop and start of the data being transmitted.

The representation of logical ones and zeroes were designed to be multiples of this basic sampling rate, defined as "r". In a preferred embodiment, one such scheme is as follows: a zero would consist of four repetitions of a sine wave (or similar pattern) that had a period of 2 * r. A one would consist of two repetitions of a sine wave (or similar pattern) that had a period of 4 * r. Therefore, each logical bit, whether zero or one, would require 8 * r time. Although the sampled time base is stable and the time base within the USB device is also similarly stable, the relative phase (sync) between these two time bases is not known. So as the audio is sampled, it is unknown at what exact phase angle within the signal the first sample will be taken. It can vary by an amount of time equal to r. Therefore, it was decided to only be concerned with zero-crossing events in the sampled signal. This then permitted the signal to be a sine wave, a square wave, or any other wave provided that it only crossed zero at two equally spaced intervals within its period. This also allowed the amplitude of the signal to vary within a fairly wide range without introducing problems and served to make the system more robust for use with inexperienced users installing inexpensive devices. Also, because there can be a small amount of noise on the signal, some amount of hysteresis processing is advantageously done to make sure that noise can't cause the appearance of extra zero-crossings.

Each signal packet is conveniently required to start with a logical one and then a zero. Furthermore, the data must be of an expected fixed length and include a checksum. Packets that don't meet this criterion are simply discarded. It is normal, given the all the uncertainties of user setup and, in a preferred embodiment, Flash® performance within multitasking Windows®, that some packets will be discarded regularly by this procedure. Notwithstanding these drawbacks, the procedure ensures that accepted packets contain accurate data with a high degree of reliability.

Taking a logical step backwards to consider data packets rather than individual logical bits and physical waveforms, the Flash® driver, as an example, is programmed to pass on lists of samples taken. Flash® itself determines how many consecutive samples are in each "list", but typically it is 2048, 4096 or 8192. The samples of audio data are then continued in the next "list" passed on by Flash® unless it is unable to keep up and do so for any of the reasons mentioned above.

In one embodiment, the driver software is programmed to monitor these lists until a list is received that has no significant audio activity at its start but does have significant audio activity at its end. It then saves a copy of that list and all successive lists received until it receives one that starts with significant audio activity and ends with no significant audio activity. If Flash® has been successful at receiving all the data without interruption contained in these lists, the concatenation of all of these lists will consist of one packet of audio data sent by the USB device. As mentioned above, these packets are of a fixed length, e.g. a few hundred bits.

At that point, having concatenated all of these lists, the application software scans the list to see at what sample significant audio activity seems to start or repeat or contain an escape sequence etc. From there, it calculates where the transition from logical one to logical zero should occur within the list and compares the plus/zero/minus characteristics of the data against what would be expected of an idealized signal. If the samples in the list meet this criteria (within an allowed sample "slide" of, currently, one position), then the following samples are counted off as per the logical bit data length described above. Each resulting logical region is then compared against the template of how a logical one would look and how a logical zero would look. If it matches one or the other (within the allowed sample "slide"), then it is considered to be successfully recognized as a logical zero or one and processing continues with further logical bits regions within the signal up to the point when the audio data level becomes insignificant. The logical ones and zeroes obtained are then considered against the criteria mentioned above (packet length and checksum), and if they meet that criteria, the data is considered legitimate and passed on for further interpretation by the application. For instance, the data might contain the serial number of the device, the moisture level or other information.

As discussed above, useful skin, scalp or hair/hair hydration transducers for evaluating skin and/or hair condition in the invention may include those adapted to measure 1) mechanical properties (such as elasticity, viscoelasticity, distensibility, indentability and the like) by way of testing techniques including but not limited to ballistometry, pressure or suction resistance, tensile strength or frictional measurement, etc.; 2) moisture properties (such as TEWL or transepidermal water loss measurement, corneometry and the like) by way of testing techniques including but not limited to hygrometry, capacitance, or conductance, etc.; 3) color properties by way of testing techniques including but not limited to reflectance or absorption photometry or spectrophotometry, etc.; 4) gloss or shine properties by way of testing techniques including but not limited to reflectometry, etc.; or any combination thereof. These transducers also produce analog signals that are or can be adjusted to be within limits compatible with hardware (70).

In a preferred embodiment, two analog sensors that are useful are a hydration sensor for measuring moisture having an external surface contactable against skin, scalp or hair including at least two adjacent metallic wires with their respective capacitance sensitive to differences in dielectric constant; and a color sensor having a plurality of light emitting diodes arranged for emitting light and also absorbing light reflected from the skin, scalp or hair area being evaluated.

Other devices that may output a digital signal conveying useful information may be used in the present invention in addition to the inventive analog transducers describe above. One such example is a digital camera including a lens system, an optical image receiver and a diffuser. The camera microchip suitably has a circuit board electrical arrangement that can adjust the lens system, store image information and transmit the images to a receiver. The optical image receiver may include a CCD or CMOS type sensor or its equivalent and the like. Other examples of useful transducers include digital versions of the analog sensors described above may be used singly or in any combination to produce more comprehensive analytical results in addition to at least one analog transducer.

While this invention has been described with respect to particular embodiments thereof, it is apparent that numerous other forms and modifications of the invention will be obvious to those skilled in the art. The appended claims and this invention generally should be construed to cover all such obvious forms and modifications which are within the true spirit and scope of the present invention.

## Claims

1. An apparatus (10) for analyzing the condition of skin, scalp or hair of a user, comprising:
a, a transducer device (20; 30) responsive to a property of the skin, scalp or hair (12) providing an analog output (32) related to the property;
b. a microcontroller (50) which is adapted to convert the transducer analog output (32) to digital values and to convert the digital values to an audio signal (52), wherein the audio signal (52) is related to the property, and c. a USB serial device comprising a USB processor (60) which is adapted to digitize the audio signal (52) to output digital signal (62), such that the analog output (32) is converted from analog to digital to analog to digital; and wherein the USB serial device (60) is adapted to transmit the digital signal (62) with a digital data transmission protocol which provides data packet transmission sufficient for an application to find both a point at which a new packet begins and an opportunity to interpret its received raw data signal, wherein the transducer device (20;30), microcontroller (50) and USB processor (60) are contained in a unitary device (40).

2. The apparatus (10) of claim 1 wherein the data packet transmission is interspersed by pauses; is interspersed by repetitive data sequences that do not appear in the serial device digital signal (62); or is interspersed by an escape sequence.

3. The apparatus (10) of claim 1 or claim 2 wherein the skin, scalp or hair property measured by the transducer (20; 30.) is selected from a mechanical property, a moisture property, a colour property, a gloss property or a combination thereof.

4. The apparatus (10,100) of anyone of the preceding claims further comprising:
a. a processor configured to receive the digital signal (62) from the serial device (60);
b. an application residing in the processor which interprets the digital signal (62);
wherein the application generates an output related to the digital signal (62) on a user interface (110); and
wherein the output is displayed as a signal lever, an image, a report, a local or remote data file or within a networked application script or a combination thereof.

5. The apparatus (10) of anyone of the preceding claims, further comprising at least one digital sensor responsive to a property of the skin, scalp or hair.

6. The apparatus (10, 100) of claim 4 further comprising a communication interface (130) connecting the user interface (110) to a remote processor wherein the remote processor generates output related to the skin, scalp or hair condition of the user.

7. The apparatus of claim 6 wherein the remote processor is connected to the communications interface (130) over a network (140).

8. A method for analyzing the condition of skin, scalp or hair (12) of a user,
comprising the steps of:
a. contacting the skin, scalp or hair (12) with a sensor coupled to a transducer device (20, 30) responsive to one or more properties of the skin, scalp or hair providing an analog output (32) related to that property;
b. transmitting the analog output from the transducer analog output to a USB serial device (60), wherein a microcontroller (50) converts the analog output (32) to digital values and converts the digital values to an audio signal (52) which is digitized by a USB processor (60);
c. outputting the digital signal (62) from the USB serial device (60); and
d. wherein the serial device (60) provides data packet transmission sufficient for an application to find both a point at which a new packet begins and an opportunity to interpret its received raw data signal wherein the transducer device (20;30), microcontroller (50) and USB processor (60) are contained in a unitary device (40); and wherein the analog output (32) is converted from analog to digital to analog to digital.

9. The method of claim 8 wherein the data packet transmission is interspersed by pauses; is interspersed by repetitive data sequences that do not appear in the serial device digital signal (62), or is interspersed by an escape sequence.

10. The method of claim 8 or claim 9 wherein the skin, scalp or hair property measured by the transducer (20, 30) is selected from a mechanical property, a moisture property, a color property, a gloss property or a combination thereof.

11. The method of anyone of claims 8 two 10 further comprising;
a. transmitting the digital signal (62) from the serial device (60) to a processor;
b. interpreting the digital signal (62) by a compatible application residing in the processor;
c. generating an output related to the digital signal (62); and
d. displaying the output as a signal level, an image, a report, a local or remote data file or within a networked application script or a combination thereof.

12. The method of anyone of claims 8 to 11 further comprising providing at least one digital sensor responsive to a property of the skin, scalp or hair.

13. The method of anyone of claims 8 to 12 further comprising providing a communication interface (130) connecting a user interface (110) to a remote processor,
wherein the remote processor generates output related to the skin, scalp or hair condition of the user.

14. The method of claim 13 wherein the remote processor is connected to the communications interface (130) over a network (140).

## Patentansprüche

1. Vorrichtung (10) zum Analysieren des Zustands von Haut, Kopfhaut oder Haar eines Benutzers, wobei die Vorrichtung Folgendes umfasst:
a. eine Signalwandler-Vorrichtung (20; 30), die auf eine Eigenschaft der Haut, der Kopfhaut oder des Haars (12) anspricht, die ein analoges Ausgangssignal (32), das sich auf die Eigenschaft bezieht, bereitstellt;
b. eine Mikrosteuerung (50), die ausgelegt ist, das analoge Signalwandler-Ausgangssignal (32) in digitale Werte umzusetzen und die digitalen Werte in ein Audiosignal (52) umzusetzen, wobei sich das Audiosignal (52) auf die Eigenschaft bezieht; und
c. eine serielle USB-Vorrichtung, die einen USB-Prozessor (60) umfasst, der ausgelegt ist, das Audiosignal (52) zu digitalisieren, um ein digitales Signal (62) auszugeben, so dass das analoge Ausgangssignal (32) von analog zu digital zu analog zu digital umgesetzt wird; und wobei die serielle USB-Vorrichtung (60) ausgelegt ist, das digitale Signal (62) mit einem Übertragungsprotokoll für digitale Signale zu übertragen, das eine Datenpaketübertragung bereitstellt, die für eine Anwendung ausreichend ist, um einen Punkt, bei dem ein neues Paket beginnt, sowie eine Gelegenheit zum Interpretieren des empfangenen Rohdatensignals zu finden, wobei die Signalwandlervorrichtung (20; 30), die Mikrosteuerung (50) und der USB-Prozessor (60) in einer einzigen Vorrichtung (40) enthalten sind.

2. Vorrichtung (10) nach Anspruch 1, wobei die Datenpaketübertragung von Pausen, von wiederholten Datensequenzen, die nicht in dem digitalen Signal (62) der seriellen Vorrichtung erscheinen oder von einer Escape-Sequenz durchsetzt ist.

3. Vorrichtung (10) nach Anspruch 1 oder 2, wobei die Haut-, Kopfhaut- oder Haareigenschaft, die von dem Signalwandler (20; 30) gemessen wird, aus einer mechanischen Eigenschaft, einer Feuchtigkeitseigenschaft, einer Farbeigenschaft, einer Glanzeigenschaft oder einer Kombination davon ausgewählt wird.

4. Vorrichtung (10, 100) nach einem der vorhergehenden Ansprüche, die ferner Folgendes umfasst:
a. einen Prozessor, der konfiguriert ist, das digitale Signal (62) von der seriellen Vorrichtung (60) zu empfangen;
b. eine Anwendung, die sich in dem Prozessor befindet, die das digitale Signal (62) interpretiert;
wobei die Anwendung bei einer Benutzerschnittstelle (110) ein Ausgangssignal erzeugt, das sich auf das digitale Signal (62) bezieht; und wobei das Ausgangssignal als eine Signalhöhe, ein Bild, ein Bericht, eine lokale oder entfernt liegende Datendatei oder in einem Netzanwendungsskript oder einer Kombination davon angezeigt wird.

5. Vorrichtung (10) nach einem der vorhergehenden Ansprüche, die ferner wenigstens einen digitalen Sensor umfasst, der auf eine Eigenschaft der Haut, der Kopfhaut oder des Haars anspricht.

6. Vorrichtung (10, 100) nach Anspruch 4, die ferner eine Kommunikationsschnittstelle (130) umfasst, die die Benutzerschnittstelle (110) mit einem entfernt liegenden Prozessor verbindet, wobei der entfernt liegende Prozessor ein Ausgangssignal erzeugt, das sich auf den Haut-, Kopfhaut- oder Haarzustand des Benutzers bezieht.

7. Vorrichtung nach Anspruch 6, wobei der entfernt liegende Prozessor mit der Kommunikationsschnittstelle (130) über ein Netz (140) verbunden ist.

8. Verfahren zum Analysieren des Zustands der Haut, der Kopfhaut oder des Haars (12) eines Benutzers, wobei das Verfahren die folgenden Schritte umfasst:
a. Berühren der Haut, der Kopfhaut oder des Haars (12) mit einem Sensor, der mit einer Signalwandlervorrichtung (20; 30) gekoppelt ist, der auf eine oder mehrere Eigenschaften der Haut, der Kopfhaut oder des Haars anspricht, wobei ein analoges Ausgangssignal (32) bereitgestellt wird, das sich auf diese Eigenschaft bezieht;
b. Übertragen des analogen Ausgangssignals von dem analogen Ausgang des Signalwandlers zu einer seriellen USB-Vorrichtung (60), wobei eine Mikrosteuerung (50) das analoge Ausgangssignal (32) in digitale Werte umsetzt und die digitalen Werte in ein Audiosignal (52) umsetzt, das durch einen USB-Prozessor (60) digitalisiert wird;
c. Ausgeben des digitalen Signals (62) von der seriellen USB-Vorrichtung (60); und
d. wobei die serielle Vorrichtung (60) eine Datenpaketübertragung bereitstellt, die für eine Anwendung ausreichend ist, um einen Punkt, bei dem ein neues Paket beginnt, sowie eine Gelegenheit zum Interpretieren der empfangenen Rohdatensignale zu finden, wobei die Signalwandlervorrichtung (20; 30), die Mikrosteuerung (50) und der USB-Prozessor (60) in einer einzigen Vorrichtung (40) enthalten sind; und wobei das analoge Ausgangssignal (32) von analog zu digital zu analog zu digital umgesetzt wird.

9. Verfahren nach Anspruch 8, wobei die Datenpaketübertragung von Pausen durchsetzt ist, von wiederholten Datensequenzen, die nicht in dem digitalen Signal (62) der seriellen Vorrichtung erscheinen, durchsetzt ist, oder von einer Escape-Sequenz durchsetzt ist.

10. Verfahren nach Anspruch 8 oder 9, wobei die Haut-, Kopfhaut- oder Haareigenschaft, die von dem Signalwandler (20; 30) gemessen wird, aus einer mechanischen Eigenschaft, einer Feuchtigkeitseigenschaft, einer Farbeigenschaft, einer Glanzeigenschaft oder einer Kombination davon ausgewählt wird.

11. Verfahren nach einem der Ansprüche 8 bis 10, das ferner die folgenden Schritte umfasst:
a. Übertragen des digitalen Signals (62) von der seriellen Vorrichtung (60) an einen Prozessor;
b. Interpretieren des digitalen Signals (62) durch eine kompatible Anwendung, die sich in dem Prozessor befindet;
c. Erzeugen eines Ausgangssignals, das sich auf das digitale Signal (62) bezieht; und
d. Anzeigen des Ausgangssignals als eine Signalhöhe, ein Bild, einen Bericht, eine lokale oder entfernt liegende Datendatei oder in einem Netzanwendungsskript oder eine Kombination davon.

12. Verfahren nach einem der Ansprüche 8 bis 11, das ferner wenigstens einen digitalen Sensor umfasst, der auf eine Eigenschaft der Haut, der Kopfhaut oder des Haars anspricht.

13. Verfahren nach einem der Ansprüche 8 bis 12, das ferner das Bereitstellen einer Kommunikationsschnittstelle (130) umfasst, die eine Benutzerschnittstelle (110) mit einem entfernt liegenden Prozessor verbindet, wobei der entfernt liegende Prozessor ein Ausgangssignal erzeugt, das sich auf den Haut-, Kopfhaut- oder Haarzustand des Benutzers bezieht.

14. Verfahren nach Anspruch 13, wobei der entfernt liegende Prozessor mit der Kommunikationsschnittstelle (130) über ein Netz (140) verbunden ist.

## Revendications

1. Appareil (10) destiné à analyser l'état de la peau, du cuir chevelu, ou des cheveux d'un utilisateur, comprenant :
a. un dispositif transducteur (20 ; 30) répondant à une propriété de la peau, du cuir chevelu, ou des cheveux (12), fournissant une sortie analogique (32) connexe à la propriété ;
b. un microcontrôleur (50) qui est apte à convertir la sortie analogique de transducteur (32) en valeurs numériques et à convertir les valeurs numériques en un signal audio (52), dans lequel le signal audio (52) est connexe à la propriété ; et
c. un dispositif USB sériel comprenant un processeur USB (60) qui est apte à numériser le signal audio (52) en vue de délivrer en sortie un signal numérique (62), de sorte que la sortie analogique (32) est convertie d'un format analogique à numérique et numérique à analogique ; et dans lequel le dispositif USB sériel (60) est apte à transmettre le signal numérique (62) au moyen d'un protocole de transmission de données numériques qui fournit une transmission de paquets de données suffisante pour permettre à une application de trouver à la fois un point auquel commence un nouveau paquet et une occasion d'interpréter son signal de données brutes reçu ; dans lequel le dispositif transducteur (20 ; 30), le microcontrôleur (50) et le processeur USB (60) sont contenus dans un dispositif unitaire (40).

2. Appareil (10) selon la revendication 1, dans lequel la transmission de paquets de données est entrecoupée par des pauses ; est entrecoupée par des séquences de données répétitives qui n'apparaissent pas dans le signal numérique de dispositif sériel (62) ; ou est entrecoupée par une séquence d'échappement.

3. Appareil (10) selon la revendication 1 ou 2, dans lequel la propriété de la peau, du cuir chevelu ou des cheveux mesurée par le transducteur (20 ; 30) est sélectionnée parmi une propriété mécanique, une propriété d'hydratation, une propriété de couleur, une propriété d'éclat ou une combinaison de cela.

4. Appareil (10, 100) selon l'une quelconque des revendications précédentes, comprenant en outre :
a. un processeur configuré de manière à recevoir le signal numérique (62) en provenance du dispositif sériel (60) ;
b. une application résidant dans le processeur qui interprète le signal numérique (62) ;
dans lequel l'application génère une sortie connexe au signal numérique (62) sur une interface utilisateur (110) ; et
dans lequel la sortie est affichée sous la forme d'un niveau de signal, d'une image, d'un rapport, d'un fichier de données local ou distant, ou dans un script d'application en réseau, ou une combinaison de cela.

5. Appareil (10) selon l'une quelconque des revendications précédentes, comprenant en outre au moins un capteur numérique répondant à une propriété de la peau, du cuir chevelu, ou des cheveux.

6. Appareil (10, 100) selon la revendication 4, comprenant en outre une interface de communication (130) connectant l'interface utilisateur (110) à un processeur distant, dans lequel le processeur distant génère une sortie connexe à l'état de la peau, du cuir chevelu, ou des cheveux de l'utilisateur.

7. Appareil selon la revendication 6, dans lequel le processeur distant est connecté à l'interface de communication (130) sur un réseau (140).

8. Procédé d'analyse de l'état de la peau, du cuir chevelu, ou des cheveux (12) d'un utilisateur, comprenant les étapes ci-dessous consistant à :
a. mettre en contact la peau, le cuir chevelu ou les cheveux (12) avec un capteur couplé à un dispositif transducteur (20 ; 30) répondant à une ou plusieurs propriétés de la peau, du cuir chevelu, ou des cheveux fournissant une sortie analogique (32) connexe à cette propriété ;
b. transmettre la sortie analogique, de la sortie analogique de transducteur à un dispositif USB sériel (60), dans lequel un microcontrôleur (50) convertit la sortie analogique (32) en des valeurs numériques et convertit les valeurs numériques en un signal audio (52) qui est numérisé par un processeur USB (60) ;
c. délivrer en sortie le signal numérique (62) à partir du dispositif USB sériel (60) ; et
d. dans lequel le dispositif sériel (60) fournit une transmission de paquets de données suffisante pour permettre à une application de trouver à la fois un point auquel commence un nouveau paquet et une occasion d'interpréter son signal de données brutes reçu ;
dans lequel le dispositif transducteur (20 ; 30), le microcontrôleur (50) et le processeur USB (60) sont contenus dans un dispositif unitaire (40) ; et
dans lequel la sortie analogique (32) est convertie d'un format analogique à numérique et numérique à analogique.

9. Procédé selon la revendication 8, dans lequel la transmission de paquets de données est entrecoupée par des pauses ; est entrecoupée par des séquences de données répétitives qui n'apparaissent pas dans le signal numérique de dispositif sériel (62) ; ou est entrecoupée par une séquence d'échappement.

10. Procédé selon la revendication 8 ou 9, dans lequel la propriété de la peau, du cuir chevelu ou des cheveux mesurée par le transducteur (20, 30) est sélectionnée parmi une propriété mécanique, une propriété d'hydratation, une propriété de couleur, une propriété d'éclat ou une combinaison de cela.

11. Procédé selon l'une quelconque des revendications 8 à 10 comprenant en outre :
a. transmettre le signal numérique (62), du dispositif sériel (60) à un processeur ;
b. interpréter le signal numérique (62) par le biais d'une application compatible résidant dans le processeur ;
c. générer une sortie connexe au signal numérique (62) ; et
d. afficher la sortie sous la forme d'un niveau de signal, d'une image, d'un rapport, d'un fichier de données local ou distant, ou dans un script d'application en réseau, ou une combinaison de cela.

12. Procédé selon l'une quelconque des revendications 8 à 11, comprenant en outre l'étape consistant à fournir au moins un capteur numérique répondant à une propriété de la peau, du cuir chevelu, ou des cheveux.

13. Procédé selon l'une quelconque des revendications 8 à 12, comprenant en outre l'étape consistant à fournir une interface de communication (130) connectant une interface utilisateur (110) à un processeur distant ;
dans lequel le processeur distant génère une sortie connexe à l'état de la peau, du cuir chevelu, ou des cheveux de l'utilisateur.

14. Procédé selon la revendication 13, dans lequel le processeur distant est connecté à l'interface de communication (130) sur un réseau (140).
